# FASCICULE DE BREVET EUROPEEN

(11) **EP 2 673 413 B1**
(45) Date de publication et mention de la délivrance du brevet: **11.04.2018**
(21) Numéro de dépôt: 12707888.9
(22) Date de dépôt: 09.02.2012
(51) Int. Cl.: D06M 23/12, D06M 23/08, A61K 9/51, A61K 8/41, A61Q 19/00

(54) **MICROPARTICULES CATIONIQUES ET UTILISATION DANS LE DOMAINE DES COSMETO-TEXTILES**
KATIONISCHEN MIKROTEILCHEN UND VERWENDUNG AUF DEM GEBIET DER KOSMETISCHEN TEXTILIEN
CATIONIC MICROPARTICLES AND USE IN THE FIELD OF COSMETOTEXTILES

(30) Priorité: 10.02.2011 FR 1151071
(43) Date de publication de la demande: 18.12.2013
(73) Titulaire: Alpol Cosmetique, 01500 Château Gaillard (FR); Université Claude Bernard, 69100 Villeurbanne (FR); Centre National de la Recherche Scientifique, 69609 Villeurbanne Cedex (FR)
(72) Inventeur: RIPOLL, Lionel, F-69100 Villeurbanne (FR); BORDES, Claire, F-69360 Saint Symphorien d'Ozon (FR); FESSI, Hatem, F-69110 Villeurbanne (FR); ELAISSARI, Abdelhamid, F-69230 Saint Genis Laval (FR); FAURE, Philippe, F-69580 Sathonay Camp (FR); ETHEVE, Sabrina, F-69100 Villeurbanne (FR)
(74) Mandataire: Agasse, Stéphane
(86) Numéro de dépôt international: PCT/FR2012/050276
(87) Numéro de publication internationale: WO 2012/107694

(56) Documents cités:
- WO-A1-01/02087
- WO-A1-01/62376
- WO-A2-2008/068418
- US-A1- 2007 116 697

## Description

La présente invention a pour objet de nouvelles microparticules cationiques, un procédé de préparation de ces particules, et leur utilisation pour la préparation de cosmétotextiles.

Les cosmétotextiles ou textiles dits « fonctionnalisés », sont des textiles contenant des substances ou des préparations cométiques, pharmaceutiques et/ou dermatologiques destinées à être libérées sur la peau humaine qui revêt ledit cosmétotextile. Selon la définition du Bureau de Normalisation des Industries Textiles et de l'Habillement (BNITH), un cosmétotextile est un article textile contenant une substance ou une préparation destinée à être libérée durablement sur les différentes parties superficielles du corps humain, notamment sur l'épiderme, et revendiquant une (ou des) propriété(s) particulière(s) telle(s) que le nettoyage, le parfumage, la modification d'aspect, la protection, le maintien en bon état ou la correction d'odeurs corporelles.

De nombreuses problématiques sont associées à l'utilisation des cosmétotextiles, au premier rang desquelles leur faible durée d'efficacité. Depuis le début des années 90, de nombreuses solutions ont ainsi été proposées pour tenter d'améliorer la qualité et la durée de vie des cosmétotextiles.

Certains cosmétotextiles, tels que ceux décrits dans la demande EP-A-1353002, sont préparés par pulvérisation sur le textile d'une ou plusieurs substances en solution ou en dispersion dans un milieu liquide de façon à réaliser un film continu qui vient se loger dans les mailles de l'article textile et ainsi en remplir les orifices. Cependant, de tels cosmétotextiles présentent le désavantage d'une faible durée de vie, le film continu ainsi formée ne résistant pas ou peu aux lavages.

D'autres cosmétotextiles sont imprégnés de particules solides, généralement des microparticules, contenant une quantité spécifique d'une substance ou d'une préparation (généralement cosmétique) destinée à être libérée sur le corps humain. La libération de la substance ou de la préparation est généralement déclenchée par un impact, habituellement un mouvement de friction ou de pression entre le corps et le tissu, provoquant la rupture des capsules en fragments et libérant ainsi ladite substance ou composition. Une libération prolongée peut également être envisagée par diffusion de la substance ou de la préparation à travers la matrice polymère de la particule ou lors de la dégradation progressive de cette matrice polymère.

Jusqu'à présent, les particules contenant la substance ou la préparation destinée à être libérée sont immobilisées sur le textile grâce à des interactions physiques (liaisons de type van der Waals ou hydrogénées) ou chimiques (liaisons covalentes).

La demande de brevet internationale WO-A-2008/068418 décrit par exemple l'utilisation de nanoparticules choisies parmi le dioxyde de titane, l'oxyde de zinc, les fullerènes, les nanocristaux, les nanoémulsions, les nanocapsules, les nanosphères, les nanovésicules et les sphérulites pour la préparation de cosmétotextile. Les nanoparticules sont accrochées au textile par imprégnation dans un bain porté à une température d'environ 40°C.

La demande de brevet internationale WO-A-01/62376 décrit quant à elle des microcapsules préparées à partir de divers polymères et contenant un agent actif tel que par exemple un agent antimicrobien. Diverses utilisations de ces microcapsules sont envisagées, notamment pour la préparation de cosmétotextiles. Le bromure d'hexadecyl trimethyl ammonium est un des nombreux agents actifs antimicrobiens cités comme pouvant être contenus dans les microcapsules décrites. En tant qu'agent actif, il ne participe cependant pas à la structure de l'enveloppe desdites microcapsules.

Les cosmétotextiles préparés à l'aide des particules décrites précédemment présentent une qualité et une durée de vie insuffisantes, principalement du fait :
- de la faible résistance au lavage et aux frottements des particules, celles-ci ayant une forte propension à être arrachées du textile;
- et de l'épuisement des particules, celles-ci se vidant plus ou moins rapidement de l'actif qu'elles contiennent.

Une des solutions envisagées pour pallier à cette faible résistance au lavage des particules et à l'épuisement de celles-ci est la possibilité de «réactiver» (ou « recharger ») les cosmétotextiles en y accrochant de nouvelles particules. Cependant, les manipulations devant être effectuées par le consommateur sont souvent fastidieuses et complexes. Ces manipulations doivent au surplus être effectuées très fréquemment (parfois de façon journalière) du fait de la faible durée de vie des microparticules délivrant l'actif. L'utilisation de dosettes à mettre dans la machine à laver afin de réactiver le cosmétotextile présente également le désavantage d'imposer un lavage uniquement pour le cosmétotextile utilisé sous peine d'activer tous les autres vêtements présents dans la machine.

Enfin, la plupart des microparticules utilisées jusqu'à présent pour la préparation des cosmétotextiles présente également le désavantage de pouvoir incorporer uniquement des actifs lipophiles à l'exclusion de tout actif hydrophile.

Ainsi, à la date de la présente invention, il demeure nécessaire de fournir des particules qui puissent être utilisées pour préparer des cosmétotextiles, et qui démontrent une meilleure résistance au lavage, qui permettent une libération prolongée des actifs qu'elles contiennent, et ce qu'ils soient hydrophiles ou lipophiles, tout en étant facilement accrochables au textile afin de permettre, le cas échéant, une « réactivation » du cosmétotextile aisée pour le consommateur.

Or, il a maintenant été trouvé des particules qui permettent de répondre efficacement à ces problématiques.

Ainsi, la présente invention a pour objet des microparticules cationiques de diamètre compris entre 1 nm et 100 µm dont l'enveloppe comprend :
- un ou plusieurs polymère(s) choisi(s) parmi la polycaprolactone, l'acide polylactique, l'éthyle cellulose, le polymethylmethacrylate et un copolymère d'acrylates et d'ammonium méthacrylate ; et
- un agent stabilisant choisi parmi le bromure d'hexadecyl trimethyl ammonium, le chlorure de benzéthonium, l'alcool polyvinylique et l'aminodextran ;
le rapport en poids agent stabilisant/polymère étant compris entre 0,001 et 0,5.

Les microparticules selon la présente invention permettent de résoudre efficacement les problématiques rencontrées dans le domaine des cosmétotextiles. En effet, les particules selon l'invention possèdent un potentiel zêta positif (c'est-à-dire des particules cationiques) qui permet d'accrocher facilement les particules au textile par le biais d'interactions électrostatiques capables de résister beaucoup plus longtemps aux contraintes imposées par les frottements et les lavages. Ainsi, les particules selon la présente invention peuvent rester accrochées au textile malgré un nombre important de lavages et d'utilisations. D'autre part, les particules selon l'invention permettent une libération prolongée de l'actif qu'elles contiennent, que celui-ci soit hydrophile ou lipophile, ce qui permet de limiter la fréquence de réactivation du cosmétotextile. Enfin, les particules selon la présente invention permettent une réactivation aisée du cosmétotextile par le consommateur, par simple trempage du textile dans un bain contenant lesdites microparticules ou par simple vaporisation d'une solution contenant lesdites microparticules.

Dans le cadre de la présente invention :
- le terme « microparticule » désigne également une microcapsule ou une microsphère;
- le terme « enveloppe » désigne la membrane de la particule permettant d'encapsuler un produit ou un ensemble des produits;
- on entend par « cosmétotextile », tout article textile contenant une substance ou une préparation destinée à être libérée durablement sur les différentes parties superficielles du corps humain, notamment sur l'épiderme, et revendiquant une (ou des) propriété(s) particulières ;
- on entend par « potentiel zêta » le potentiel zêta des particules mesuré à 25°C dans une solution à 1 mmol de NaCl;
- on entend par « réactivation » (ou « réactiver ») tout procédé pouvant être mis en oeuvre par le consommateur permettant d'accrocher au cosmétotextile de nouvelles particules contenant un actif ;
- on entend par « actif » toute substance ou préparation à usage cosmétique, pharmaceutique et/ou dermocosmétique, ainsi que les parfums ;
- polycaprolactone (ou PCL) désigne le composé enregistré sous le numéro CAS 24980-41-4 ;
- acide polylactique (ou PLA) désigne le composé enregistré sous le numéro CAS 26100-51-6 ;
- éthyle cellulose (ou EC) désigne le composé enregistré sous le numéro CAS 9004-57-3;
- polymethylmethacrylate (ou PMMA) désigne le composé enregistré sous le numéro CAS 9011-14-7 ;
- « copolymère d'acide méthacrylique et de méthylméthacrylate » désigne en particulier le composé ayant pour nom IUPAC poly(ethyl acrylate-co-methyl methacrylate-co-trimethylammonioethyl methacrylate chloride) 1:2:0.1, enregistré sous le numéro CAS 33434-24-1 et commercialisé par la société Rohm & Haas sous le nom Eudragit® RS 100 ;
- bromure d'hexadecyl trimethyl ammonium (ou CTAB) désigne le composé enregistré sous le numéro CAS 57-09-0 ;
- chlorure de benzéthonium (ou BZC) désigne le composé enregistré sous le numéro CAS 121-54-0 ;
- alcool polyvinylique (ou PVA) désigne le composé enregistré sous le numéro CAS 9002-89-5.

Préférentiellement, la présente invention a pour objet des microparticules dans lesquelles les caractéristiques suivantes sont choisies seules ou en combinaison :
- le diamètre des particules est compris entre 100 nm et 1000 nm, de préférence encore entre 150 nm et 800 nm;
- le polymère est choisi comme étant la polycaprolactone, le polymethylmethacrylate, un copolymère d'acrylates et d'ammonium méthacrylate, une association polymethylmethacrylate/polycaprolactone dans un rapport en poids polymethylmethacrylate/polycaprolactone compris entre 1 et 10, une association polymethylmethacrylate/acide polylactique dans un rapport en poids polymethylmethacrylate/acide polylactique compris entre 1 et 10, une association polymethylmethacrylate/éthyle cellulose dans un rapport en poids polymethylmethacrylate/éthyle cellulose compris entre 1 et 10, ou une association polymethylmethacrylate/copolymère d'acrylates et d'ammonium méthacrylate dans un rapport en poids polymethylmethacrylate/copolymère d'acrylates et d'ammonium méthacrylate compris entre 1 et 10. De préférence encore, le polymère est choisi comme étant le polymethylmethacrylate ou une association polymethylmethacrylate/polycaprolactone dans un rapport en poids polymethylmethacrylate/polycaprolactone compris entre 1 et 10 ;
- l'agent stabilisant est choisi comme étant le bromure d'hexadecyl trimethyl ammonium ou le chlorure de benzéthonium ; et/ou
- le rapport en poids agent stabilisant/polymère est compris entre 0,005 et 0,1 ; de préférence encore entre 0,01 et 0,05.

Comme exemples de microparticules selon la présente invention, on peut notamment citer les microparticules dont l'enveloppe comprend :
- la polycaprolactone et le bromure d'hexadecyl trimethyl ammonium ;
- la polycaprolactone et le chlorure de benzéthonium ;
- la polycaprolactone et l'alcool polyvinylique ;
- la polycaprolactone et l'aminodextran ;
- l'acide polylactique et le bromure d'hexadecyl trimethyl ammonium ;
- l'acide polylactique et le chlorure de benzéthonium ;
- l'acide polylactique et l'alcool polyvinylique ;
- l'acide polylactique et l'aminodextran ;
- l'éthyle cellulose et le bromure d'hexadecyl trimethyl ammonium ;
- l'éthyle cellulose et le chlorure de benzéthonium ;
- l'éthyle cellulose et l'alcool polyvinylique ;
- l'éthyle cellulose et l'aminodextran ;
- le polymethylmethacrylate et le bromure d'hexadecyl trimethyl ammonium ;
- le polymethylmethacrylate et le chlorure de benzéthonium ;
- le polymethylmethacrylate et l'alcool polyvinylique ;
- le polymethylmethacrylate et l'aminodextran ;
- un copolymère d'acrylates et d'ammonium méthacrylate et le bromure d'hexadecyl trimethyl ammonium ;
- un copolymère d'acrylates et d'ammonium méthacrylate et le chlorure de benzéthonium ;
- un copolymère d'acrylates et d'ammonium méthacrylate et l'alcool polyvinylique ;
- un copolymère d'acrylates et d'ammonium méthacrylate et l'aminodextran ;
- une association polymethylmethacrylate/polycaprolactone dans un rapport en poids polymethylmethacrylate/polycaprolactone compris entre 1 et 10 et le bromure d'hexadecyl trimethyl ammonium ;
- une association polymethylmethacrylate/polycaprolactone dans un rapport en poids polymethylmethacrylate/polycaprolactone compris entre 1 et 10 et le chlorure de benzéthonium ;
- une association polymethylmethacrylate/polycaprolactone dans un rapport en poids polymethylmethacrylate/polycaprolactone compris entre 1 et 10 et l'alcool polyvinylique ;
- une association polymethylmethacrylate/polycaprolactone dans un rapport en poids polymethylmethacrylate/polycaprolactone compris entre 1 et 10 et l'aminodextran ;
- une association polymethylmethacrylate/acide polylactique dans un rapport en poids polymethylmethacrylate/acide polylactique compris entre 1 et 10 et le bromure d'hexadecyl trimethyl ammonium ;
- une association polymethylmethacrylate/acide polylactique dans un rapport en poids polymethylmethacrylate/acide polylactique compris entre 1 et 10 et le chlorure de benzéthonium ;
- une association polymethylmethacrylate/acide polylactique dans un rapport en poids polymethylmethacrylate/acide polylactique compris entre 1 et 10 et l'alcool polyvinylique ;
- une association polymethylmethacrylate/acide polylactique dans un rapport en poids polymethylmethacrylate/acide polylactique compris entre 1 et 10 et l'aminodextran ;
- une association polymethylmethacrylate/éthyle cellulose dans un rapport en poids polymethylmethacrylate/éthyle cellulose compris entre 1 et 10 et le bromure d'hexadecyl trimethyl ammonium ;
- une association polymethylmethacrylate/éthyle cellulose dans un rapport en poids polymethylmethacrylate/éthyle cellulose compris entre 1 et 10 et le chlorure de benzéthonium ;
- une association polymethylmethacrylate/éthyle cellulose dans un rapport en poids polymethylmethacrylate/éthyle cellulose compris entre 1 et 10 et l'alcool polyvinylique ;
- une association polymethylmethacrylate/éthyle cellulose dans un rapport en poids polymethylmethacrylate/éthyle cellulose compris entre 1 et 10 et l'aminodextran ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10 et le bromure d'hexadecyl trimethyl ammonium ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10 et le chlorure de benzéthonium ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10 et l'alcool polyvinylique ; et
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10 et l'aminodextran.

Comme exemples préférés de microparticules selon la présente invention, on peut citer les microparticules dont l'enveloppe comprend :
- la polycaprolactone et le bromure d'hexadecyl trimethyl ammonium ;
- la polycaprolactone et le chlorure de benzéthonium ; et
- le polymethylmethacrylate et l'alcool polyvinylique.

Dans certains cas, il peut être souhaitable que l'enveloppe des microparticules selon la présente invention contienne en plus un second agent stabilisant désigné par « agent co-stabilisant ». Ainsi, la présente invention a également pour objet des microcapsules telle que décrites précédemment dont l'enveloppe comprend en plus un agent co-stabilisant choisi parmi le bromure d'hexadecyl trimethyl ammonium, le chlorure de benzéthonium, l'alcool polyvinylique et l'aminodextran, ledit agent co-stabilisant étant différent de l'agent stabilisant.

Comme exemples d'association agent stabilisant/agent co-stabilisant selon la présente invention, on peut citer les associations suivantes :
- le bromure d'hexadecyl trimethyl ammonium et le chlorure de benzéthonium ;
- le bromure d'hexadecyl trimethyl ammonium et l'alcool polyvinylique ;
- le bromure d'hexadecyl trimethyl ammonium et l'aminodextran ;
- le chlorure de benzéthonium et l'alcool polyvinylique ;
- le chlorure de benzéthonium et l'aminodextran ; et
- l'alcool polyvinylique et l'aminodextran.

Comme exemples préférés d'association agent stabilisant/agent co-stabilisant selon la présente invention, on peut citer :
- le bromure d'hexadecyl trimethyl ammonium et l'alcool polyvinylique ; et
- le chlorure de benzéthonium et l'alcool polyvinylique.

Ainsi, comme exemples de microparticules selon la présente invention, on peut également citer les microparticules dont l'enveloppe comprend :
- la polycaprolactone, le bromure d'hexadecyl trimethyl ammonium et le chlorure de benzéthonium ;
- la polycaprolactone, le bromure d'hexadecyl trimethyl ammonium et l'alcool polyvinylique ;
- la polycaprolactone, le bromure d'hexadecyl trimethyl ammonium et l'aminodextran ;
- la polycaprolactone, le chlorure de benzéthonium et l'alcool polyvinylique ;
- la polycaprolactone, le chlorure de benzéthonium et l'aminodextran ;
- la polycaprolactone, l'alcool polyvinylique et l'aminodextran ;
- l'acide polylactique, le bromure d'hexadecyl trimethyl ammonium et le chlorure de benzéthonium ;
- l'acide polylactique, le bromure d'hexadecyl trimethyl ammonium et l'alcool polyvinylique ;
- l'acide polylactique, le bromure d'hexadecyl trimethyl ammonium et l'aminodextran ;
- l'acide polylactique, le chlorure de benzéthonium et l'alcool polyvinylique ;
- l'acide polylactique, le chlorure de benzéthonium et l'aminodextran ;
- l'acide polylactique, l'alcool polyvinylique et l'aminodextran ;
- l'éthyle cellulose, le bromure d'hexadecyl trimethyl ammonium et le chlorure de benzéthonium ;
- l'éthyle cellulose, le bromure d'hexadecyl trimethyl ammonium et l'alcool polyvinylique ;
- l'éthyle cellulose, le bromure d'hexadecyl trimethyl ammonium et l'aminodextran ;
- l'éthyle cellulose, le chlorure de benzéthonium et l'alcool polyvinylique ;
- l'éthyle cellulose, le chlorure de benzéthonium et l'aminodextran ;
- l'éthyle cellulose, l'alcool polyvinylique et l'aminodextran ;
- le polymethylmethacrylate, le bromure d'hexadecyl trimethyl ammonium et le chlorure de benzéthonium ;
- le polymethylmethacrylate, le bromure d'hexadecyl trimethyl ammonium et l'alcool polyvinylique ;
- le polymethylmethacrylate, le bromure d'hexadecyl trimethyl ammonium et l'aminodextran ;
- le polymethylmethacrylate, le chlorure de benzéthonium et l'alcool polyvinylique ;
- le polymethylmethacrylate, le chlorure de benzéthonium et l'aminodextran ;
- le polymethylmethacrylate, l'alcool polyvinylique et l'aminodextran ;
- un copolymère d'acrylates et d'ammonium méthacrylate, le bromure d'hexadecyl trimethyl ammonium et le chlorure de benzéthonium ;
- un copolymère d'acrylates et d'ammonium méthacrylate, le bromure d'hexadecyl trimethyl ammonium et l'alcool polyvinylique ;
- un copolymère d'acrylates et d'ammonium méthacrylate, le bromure d'hexadecyl trimethyl ammonium et l'aminodextran ;
- un copolymère d'acrylates et d'ammonium méthacrylate, le chlorure de benzéthonium et l'alcool polyvinylique ;
- un copolymère d'acrylates et d'ammonium méthacrylate, le chlorure de benzéthonium et l'aminodextran ;
- un copolymère d'acrylates et d'ammonium méthacrylate, l'alcool polyvinylique et l'aminodextran ;
- une association polymethylmethacrylate/polycaprolactone dans un rapport en poids polymethylmethacrylate/polycaprolactone compris entre 1 et 10, le bromure d'hexadecyl trimethyl ammonium et le chlorure de benzéthonium ;
- une association polymethylmethacrylate/polycaprolactone dans un rapport en poids polymethylmethacrylate/polycaprolactone compris entre 1 et 10, le bromure d'hexadecyl trimethyl ammonium et l'alcool polyvinylique ;
- une association polymethylmethacrylate/polycaprolactone dans un rapport en poids polymethylmethacrylate/polycaprolactone compris entre 1 et 10, le bromure d'hexadecyl trimethyl ammonium et l'aminodextran ;
- une association polymethylmethacrylate/polycaprolactone dans un rapport en poids polymethylmethacrylate/polycaprolactone compris entre 1 et 10, le chlorure de benzéthonium et l'alcool polyvinylique ;
- une association polymethylmethacrylate/polycaprolactone dans un rapport en poids polymethylmethacrylate/polycaprolactone compris entre 1 et 10, le chlorure de benzéthonium et l'aminodextran ;
- une association polymethylmethacrylate/polycaprolactone dans un rapport en poids polymethylmethacrylate/polycaprolactone compris entre 1 et 10, l'alcool polyvinylique et l'aminodextran ;
- une association polymethylmethacrylate/acide polylactique dans un rapport en poids polymethylmethacrylate/ acide polylactique compris entre 1 et 10, le bromure d'hexadecyl trimethyl ammonium et le chlorure de benzéthonium ;
- une association polymethylmethacrylate/acide polylactique dans un rapport en poids polymethylmethacrylate/ acide polylactique compris entre 1 et 10, le bromure d'hexadecyl trimethyl ammonium et l'alcool polyvinylique ;
- une association polymethylmethacrylate/acide polylactique dans un rapport en poids polymethylmethacrylate/ acide polylactique compris entre 1 et 10, le bromure d'hexadecyl trimethyl ammonium et l'aminodextran ;
- une association polymethylmethacrylate/acide polylactique dans un rapport en poids polymethylmethacrylate/ acide polylactique compris entre 1 et 10, le chlorure de benzéthonium et l'alcool polyvinylique ;
- une association polymethylmethacrylate/acide polylactique dans un rapport en poids polymethylmethacrylate/ acide polylactique compris entre 1 et 10, le chlorure de benzéthonium et l'aminodextran ;
- une association polymethylmethacrylate/acide polylactique dans un rapport en poids polymethylmethacrylate/ acide polylactique compris entre 1 et 10, l'alcool polyvinylique et l'aminodextran ;
- une association polymethylmethacrylate/éthyle cellulose dans un rapport en poids polymethylmethacrylate/éthyle cellulose compris entre 1 et 10, le bromure d'hexadecyl trimethyl ammonium et le chlorure de benzéthonium ;
- une association polymethylmethacrylate/éthyle cellulose dans un rapport en poids polymethylmethacrylate/éthyle cellulose compris entre 1 et 10, le bromure d'hexadecyl trimethyl ammonium et l'alcool polyvinylique ;
- une association polymethylmethacrylate/éthyle cellulose dans un rapport en poids polymethylmethacrylate/éthyle cellulose compris entre 1 et 10, le bromure d'hexadecyl trimethyl ammonium et l'aminodextran ;
- une association polymethylmethacrylate/éthyle cellulose dans un rapport en poids polymethylmethacrylate/éthyle cellulose compris entre 1 et 10, le chlorure de benzéthonium et l'alcool polyvinylique ;
- une association polymethylmethacrylate/éthyle cellulose dans un rapport en poids polymethylmethacrylate/éthyle cellulose compris entre 1 et 10, le chlorure de benzéthonium et l'aminodextran ;
- une association polymethylmethacrylate/éthyle cellulose dans un rapport en poids polymethylmethacrylate/éthyle cellulose compris entre 1 et 10, l'alcool polyvinylique et l'aminodextran ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10, le bromure d'hexadecyl trimethyl ammonium et le chlorure de benzéthonium ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10, le bromure d'hexadecyl trimethyl ammonium et l'alcool polyvinylique ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10, le bromure d'hexadecyl trimethyl ammonium et l'aminodextran ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10, le chlorure de benzéthonium et l'alcool polyvinylique ;
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10, le chlorure de benzéthonium et l'aminodextran ; et
- une association polymethylmethacrylate/copolymère d'acrylates et d'ammonium méthacrylate dans un rapport en poids polymethylmethacrylate/copolymère d'acrylates et d'ammonium méthacrylate compris entre 1 et 10, l'alcool polyvinylique et l'aminodextran.

Les microparticules selon la présente invention possèdent un potentiel zêta positif. De façon préférée, les microparticules selon la présente invention sont choisies de sorte que leur potentiel zêta soit supérieur ou égal à 10 mV. De préférence encore, les microparticules selon la présente invention sont choisies de sorte que leur potentiel zêta soit supérieur ou égal à 20 mV. De façon toute à fait préférée les microparticules selon la présente invention sont choisies de sorte que leur potentiel zêta soit supérieur ou égal à 30 mV.

L'enveloppe des microparticules selon l'invention peut également contenir tout additif conventionnellement utilisé tels que des agents stabilisants, des tensio-actifs, des émulsionnants et/ou des co-émulsionnants.

La présente invention a également pour objet un procédé de préparation de microparticules telles que décrites précédemment. Le procédé de préparation des microparticules peut différer selon que l'actif incorporé dans les microparticules objet de la présente invention est lipophile ou hydrophile. Ainsi, dans le cas d'un actif lipophile, la présente invention a pour objet un procédé de préparation de microparticules telles que décrites précédemment comprenant les étapes suivantes :
- préparation d'une solution organique par dissolution du polymère décrit précédemment et de l'actif lipophile dans un solvant organique, tel que le dichlorométhane, l'acétate d'éthyle ou l'acétone ;
- préparation d'une solution aqueuse contenant l'agent stabilisant tel que défini précédemment ;
- mise en contact de la solution organique et de la solution aqueuse ainsi préparées sous agitation durant le temps nécessaire à la formation de l'émulsion ;
- dilution de l'émulsion ainsi obtenue dans un grand volume d'eau, typiquement 2 à 5 fois le volume de l'émulsion, jusqu'à évaporation complète du solvant organique.

Dans le cas d'un actif lipophile, un procédé polymérisation radicalaire peut également être envisagé pour préparer les microparticules selon la présente invention. Ainsi, la présente invention a pour objet un procédé de préparation de microparticules telles que décrites précédemment comprenant les étapes suivantes :
- préparation d'une solution organique contenant le ou les monomère(s) nécessaires à la préparation du polymère décrit précédemment et l'actif lipophile ;
- préparation d'une solution aqueuse contenant l'agent stabilisant tel que défini précédemment et un amorceur de polymérisation tel que par exemple le 2,2'-azobis(2-methylpropionamidine) dihydrochloride, le 2,2'-azobis[N-(2-carboxyethyl)-2-methylpropionamidine]hydrate, le 2,2'-Azobis(1-imino-1-pyrrolidino-2-ethylpropane)dihydrochloride, le 2,2'-azobis(N-butyl-2-methylpropionamide), le 2,2'-azobis[N-(2-propenyl)-2-methylpropionamide], le 2,2'-Azobis(2-methylbutyronitrile), ou le bis(alkylphenyl) iodonium hexafluorophosphate ;
- mise en contact de la solution organique et de la solution aqueuse ainsi préparées sous agitation durant le temps nécessaire à la formation de l'émulsion.

Dans le cas d'un actif hydrophile, un procédé dit de « triple émulsion » peut être utilisé pour préparer les microparticules selon la présente invention. Ainsi, la présente invention a également pour objet un procédé de préparation de microparticules telles que décrites précédemment comprenant les étapes suivantes :
- préparation d'une solution aqueuse (1) par solubilisation de l'actif hydrophile dans une solution aqueuse contenant un gent stabilisant tel que défini précédemment ;
- préparation d'une solution aqueuse (2) contenant un agent stabilisant tel que défini précédemment, ledit agent stabilisant pouvant être identique ou différent de celui contenu dans la solution aqueuse (1) ;
- préparation d'une solution organique par dissolution du polymère décrit précédemment dans un solvant organique tel que le dichlorométhane, l'acétate d'éthyle ou l'acétone ;
- mise en contact de la solution aqueuse (1) et de la solution organique ainsi préparées sous agitation vive durant le temps nécessaire à la formation d'une émulsion (1).
- mise en contact de l'émulsion (1) ainsi préparée avec la solution aqueuse (2) sous agitation vive pendant le temps nécessaire à la formation de l'émulsion (2) ;
- dilution de l'émulsion (2) ainsi obtenue dans un grand volume d'eau, typiquement 2 à 5 fois le volume de l'émulsion, jusqu'à évaporation complète du solvant organique.

Les microparticules selon la présente invention peuvent contenir aussi bien des actifs hydrophiles que des actifs lipophiles.

Comme exemple d'actifs ayant des propriétés cosmétiques on peut citer les actifs anti-âges, antirides, antitaches, effet frais, effet chaud, hydratants, amincissants, anti-peau d'orange, anti-vergetures, anti-jambes lourdes, anti-repousse poil, auto-bronzants, anti-transpirants, antiperspirants ou répulsifs (« repellent »).

Comme exemples d'actifs ayant des propriétés pharmaceutiques on peut citer les actifs anti-dermatite, anti-rosacée, anti-acné, anti-psoriasis, anti-vitiligo, anti-eczéma, anti-oedème, anti-ulcère, veinotoniques, cicatrisants, antimicrobiens, virucides, anti-hématome ou antidouleurs.

Comme exemples d'actifs ayant des propriétés dermocosmétiques on peut citer les actifs anti-échauffement, anti-sécheresse, anti-desquamant, anti-rougeur ou cicatrisant.

Les microparticules selon l'invention peuvent également contenir tout additif conventionnellement utilisés tels que des conservateurs, des antioxydants, des filtres UV et/ou des solubilisants de principe actif.

Les actifs peuvent être incorporés dans les microparticules selon la présente invention par tout moyen connu de l'homme du métier parmi lesquels on peut citer l'émulsion diffusion o/w, l'émulsion diffusion w/o/w, la polymérisation radicalaire ou polymérisation en émulsion.

Les microparticules selon la présente invention, lorsqu'elles contiennent un actif tel que décrit précédemment, sont donc particulièrement utiles à la préparation de cosmétotextiles de haute qualité et ayant une très bonne durée de vie. Ainsi, la présente invention a également pour objet un article textile comprenant au moins une microparticule telle que décrite précédemment, ladite microparticule contenant elle-même un actif tel que décrit précédemment.

De préférence, les articles textiles utilisés sont choisis parmi les collants, les chaussettes, les bas, les sous-vêtements pour femme, les sous-vêtements pour homme, les sous-vêtements pour enfant, les tee-shirts, les pantalons, les leggings, les écharpes, les foulards, les gants ou le linge de maison.

Les cosmétotextiles selon la présente invention peuvent être préparés par tout moyen connu de l'homme du métier, parmi lesquels on peut citer le trempage dans un bain contenant lesdites microparticules, la vaporisation d'une solution contenant lesdites microparticules, le foulardage, le jigger ou l'enclave.

Dans le cas du trempage, on pourra par exemple faire tremper le textile, éventuellement sous agitation, dans la solution de particule à 25°C pendant 2h, puis le laisser sécher à température ambiante.

Dans le cas de la vaporisation, on pourra par exemple pulvériser la solution sur les zones à traiter du textile, puis laisser sécher à température ambiante.

On pourra accrocher les particules selon la présente invention sur toute la surface de l'article textile, lesdites particules pouvant toutes contenir le même actif ou contenir des actifs différents.

On pourra également accrocher les particules selon la présente invention contenant l'actif sur des parties délimitées de l'article textile.

Les cosmétotextiles ainsi préparés sont de haute qualité et sont plus résistants (en particulier aux lavages) que les cosmétotextiles disponibles jusqu'à présent. D'autre part, les cosmétotextiles selon l'invention permettent une libération prolongée de l'actif qu'ils contiennent, que celui-ci soit hydrophile ou lipophile, ce qui permet de limiter la fréquence de réactivation du cosmétotextile. Enfin, les cosmétotextiles selon la présente invention peuvent être aisément réactivés par le consommateur, par simple trempage du textile dans un bain contenant lesdites microparticules ou par simple vaporisation d'une solution contenant lesdites microparticules.

Ainsi, pour permettre au consommateur de réactiver leur cosmétotextile, les particules selon la présente invention peuvent prendre toute forme adéquate, telle que par exemple une solution, une poudre ou une tablette, chacune de ces formes étant éventuellement incorporée dans un sachet hydrosoluble. Ainsi, la présente invention a également pour objet une solution, une poudre ou une tablette comprenant au moins une microparticule selon la présente invention, ladite microparticule contenant elle-même un actif tel que décrit précédemment.

## Revendications

1. Microparticule cationique de diamètre compris entre 1 nm et 100 µm dont l'enveloppe comprend :
- un ou plusieurs polymère(s) choisi(s) parmi la polycaprolactone, l'acide polylactique, l'éthyle cellulose, le polymethylmethacrylate et un copolymère d'acrylates et d'ammonium méthacrylate ; et
- un agent stabilisant choisi parmi le bromure d'hexadecyl trimethyl ammonium, le chlorure de benzéthonium, l'alcool polyvinylique et l'aminodextran ;
le rapport en poids agent stabilisant/polymère étant compris entre 0,001 et 0,5.

2. Microparticule selon la revendication 1, **caractérisée en ce que** le diamètre est compris entre 150 nm et 800 nm.

3. Microparticule selon la revendication 1 ou 2, **caractérisée en ce que** le polymère est choisi comme étant le polymethylmethacrylate ou une association polymethylmethacrylate/polycaprolactone dans un rapport en poids polymethylmethacrylate/polycaprolactone compris entre 1 et 10.

4. Microparticule selon l'une des revendications 1 à 3, **caractérisée en ce que** l'agent stabilisant est choisi comme étant le bromure d'hexadecyl trimethyl ammonium ou le chlorure de benzéthonium.

5. Microparticule selon l'une des revendications 1 à 4, **caractérisée en ce que** le rapport en poids agent stabilisant/polymère est compris entre 0,005 et 0,1.

6. Microparticule selon l'une des revendications 1 à 5, **caractérisée en ce que** l'enveloppe comprend en plus un agent co-stabilisant choisi parmi le bromure d'hexadecyl trimethyl ammonium, le chlorure de benzéthonium, l'alcool polyvinylique et l'aminodextran, ledit agent co-stabilisant étant différent de l'agent stabilisant.

7. Microparticule selon l'une des revendications 1 à 6, **caractérisée en ce que** son potentiel zêta est supérieur ou égal à 30 mV.

8. Microparticule selon l'une des revendications 1 à 7, **caractérisée en ce qu'**elle contient un actif lipophile ou hydrophile.

9. Procédé de préparation de microparticules selon la revendication 8 comprenant les étapes suivantes :
- préparation d'une solution organique par dissolution du polymère décrit précédemment et de l'actif lipophile dans un solvant organique ;
- préparation d'une solution aqueuse contenant l'agent stabilisant tel que défini précédemment ;
- mise en contact de la solution organique et de la solution aqueuse ainsi préparées sous agitation durant le temps nécessaire à la formation de l'émulsion ;
- dilution de l'émulsion ainsi obtenue dans un grand volume d'eau jusqu'à évaporation complète du solvant organique.

10. Procédé de préparation de microparticules selon la revendication 8 comprenant les étapes suivantes :
- préparation d'une solution organique contenant le ou les monomère(s) nécessaires à la préparation du polymère décrit précédemment et l'actif lipophile ;
- préparation d'une solution aqueuse contenant l'agent stabilisant tel que défini précédemment et un amorceur de polymérisation ;
- mise en contact de la solution organique et de la solution aqueuse ainsi préparées sous agitation durant le temps nécessaire à la formation de l'émulsion.

11. Procédé de préparation de microparticules selon la revendication 8 comprenant les étapes suivantes :
- préparation d'une solution aqueuse (1) par solubilisation de l'actif hydrophile dans une solution aqueuse contenant un gent stabilisant tel que défini précédemment ;
- préparation d'une solution aqueuse (2) contenant un agent stabilisant tel que défini précédemment, ledit agent stabilisant pouvant être identique ou différent de celui contenu dans la solution aqueuse (1) ;
- préparation d'une solution organique par dissolution du polymère décrit précédemment dans un solvant organique tel que le dichlorométhane, l'acétate d'éthyle ou l'acétone ;
- mise en contact de la solution aqueuse (1) et de la solution organique ainsi préparées sous agitation vive durant le temps nécessaire à la formation d'une émulsion (1).
- mise en contact de l'émulsion (1) ainsi préparée avec la solution aqueuse (2) sous agitation vive pendant le temps nécessaire à la formation de l'émulsion (2) ;
- dilution de l'émulsion (2) ainsi obtenue dans un grand volume d'eau jusqu'à évaporation complète du solvant organique.

12. Utilisation de microparticules selon la revendication 8 pour la préparation d'un cosmétotextile.

13. Article textile comprenant au moins une microparticule selon la revendication 8.

14. Solution comprenant au moins une microparticule selon la revendication 8.

15. Poudre comprenant une microparticule selon la revendication 8.

16. Tablette comprenant une microparticule selon la revendication 8.

## Patentansprüche

1. Kationisches Mikropartikel mit einem Durchmesser im Bereich von 1 nm und 100 µm, von dem die Hülle Folgendes umfasst:
- ein oder mehrere Polymer(e), ausgewählt aus Polycaprolacton, Polymilchsäure, Ethylcellulose, Polymethylmethacrylat und einem Copolymer aus Acrylaten und Ammoniummethacrylat; und
- ein Stabilisierungsmittel, ausgewählt aus Hexadecyltrimethylammoniumbromid, Benzethoniumchlorid, Polyvinylalkohol und Aminodextran;
wobei das Gewichtsverhältnis von Stabilisierungsmittel/Polymer im Bereich von 0,001 und 0,5 liegt.

2. Mikropartikel gemäß Anspruch 1, **dadurch gekennzeichnet, dass** der Durchmesser im Bereich von 150 nm und 800 nm liegt.

3. Mikropartikel gemäß Anspruch 1 oder 2, **dadurch gekennzeichnet, dass** das Polymer ausgewählt ist als Polymethylmethacrylat oder eine Vereinigung von Polymethylmethacrylat/Polycaprolacton in einem Gewichtsverhältnis von Polymethylmethacrylat/Polycaprolacton im Bereich von 1 und 10.

4. Mikropartikel gemäß einem der Ansprüche 1 bis 3, **dadurch gekennzeichnet, dass** das Stabilisierungsmittel ausgewählt ist als Hexadecyltrimethylammoniumbromid oder Benzethoniumchlorid.

5. Mikropartikel gemäß einem der Ansprüche 1 bis 4, **dadurch gekennzeichnet, dass** das Gewichtsverhältnis Stabilisierungsmittel/Polymer im Bereich von 0,005 und 0,1 liegt.

6. Mikropartikel gemäß einem der Ansprüche 1 bis 5, **dadurch gekennzeichnet, dass** die Hülle des Weiteren ein Co-Stabilisierungsmittel umfasst, ausgewählt aus Hexadecyltrimethylammoniumbromid, Benzethoniumchlorid, Polyvinylalkohol und Aminodextran, wobei das Co-Stabilisierungsmittel sich von dem Stabilisierungsmittel unterscheidet.

7. Mikropartikel gemäß einem der Ansprüche 1 bis 6, **dadurch gekennzeichnet, dass** das Zeta-Potential größer oder gleich 30 mV ist.

8. Mikropartikel gemäß einem der Ansprüche 1 bis 7, **dadurch gekennzeichnet, dass** es einen lipophilen oder hydrophilen Wirkstoff enthält.

9. Zubereitungsverfahren von Mikropartikeln gemäß Anspruch 8, umfassend die folgenden Schritte:
- Zubereitung einer organischen Lösung durch Auflösung des vorstehend beschriebenen Polymers und des lipophilen Wirkstoffs in einem organischen Lösemittel;
- Zubereitung einer wässrigen Lösung, die das Stabilisierungsmittel wie vorstehend definiert enthält;
- In-Kontakt-Bringen unter Rühren der so zubereiteten organischen Lösung und der so zubereiteten wässrigen Lösung während der zum Bilden der Emulsion benötigten Zeit;
- Verdünnen der so erhaltenen Emulsion in einem großen Volumen Wasser bis zur vollständigen Verdampfung des organischen Lösemittels.

10. Zubereitungsverfahren von Mikropartikeln gemäß Anspruch 8, umfassend die folgenden Schritte:
- Zubereitung einer organischen Lösung, die das oder die zur Zubereitung des vorstehend beschriebenen Polymers notwendige(n) Monomer(e) und den lipophilen Wirkstoff enthält;
- Zubereitung einer wässrigen Lösung, die das Stabilisierungsmittel wie vorstehend definiert und einen Polymerisationsinitiator enthält;
- In-Kontakt-Bringen unter Rühren der so zubereiteten organischen Lösung und der so zubereiteten wässrigen Lösung während der zum Bilden der Emulsion benötigten Zeit.

11. Zubereitungsverfahren von Mikropartikeln gemäß Anspruch 8, umfassend die folgenden Schritte:
- Zubereitung einer wässrigen Lösung (1) durch Solubilisieren des hydrophilen Wirkstoffs in einer wässrigen Lösung, die ein Stabilisierungsmittel wie vorstehend definiert enthält;
- Zubereitung einer wässrigen Lösung (2), die ein Stabilisierungsmittel wie vorstehend definiert enthält, wobei das Stabilisierungsmittel zu dem in der wässrigen Lösung (1) enthaltenen identisch oder verschieden sein kann;
- Zubereitung einer organischen Lösung durch Auflösung des vorstehend beschriebenen Polymers in einem organischen Lösemittel wie Dichlormethan, Ethylacetat oder Aceton;
- In-Kontakt-Bringen unter lebhaftem Rühren der so zubereiteten wässrigen Lösung (1) und der so zubereiteten organischen Lösung während der zum Bilden einer Emulsion (1) benötigten Zeit.
- In-Kontakt-Bringen unter lebhaftem Rühren der so zubereiteten Emulsion (1) mit der so zubereiteten wässrigen Lösung (2) während der zum Bilden der Emulsion (2) benötigten Zeit;
- Verdünnen der so erhaltenen Emulsion (2) in einem großen Volumen Wasser bis zur vollständigen Verdampfung des organischen Lösemittels.

12. Verwendung von Mikropartikeln gemäß Anspruch 8 für die Zubereitung einer Kosmetiktextilie.

13. Textilartikel, umfassend mindestens ein Mikropartikel gemäß Anspruch 8.

14. Lösung, umfassend mindestens ein Mikropartikel gemäß Anspruch 8.

15. Pulver, umfassend ein Mikropartikel gemäß Anspruch 8.

16. Tablette, umfassend mindestens ein Mikropartikel gemäß Anspruch 8.

## Claims

1. Cationic microparticle of a diameter comprised between 1 nm and 100 µm whose shell comprises:
- one or more polymer(s) chosen among the polycaprolactone, the polylactic acid, the ethyl cellulose, the polymethyl methacrylate and a copolymer of acrylate and ammonium methacrylate; and
- a stabilizing agent chosen among the hexadecyltrimethylammonium bromide, the benzethonium chloride, the polyvinyl alcohol and the amino-dextran;
the stabilizing agent/polymer weight ratio being comprised between 0.001 and 0.5.

2. Microparticle according to claim 1, **characterized in that** the diameter is comprised between 150 nm and 800 nm.

3. Microparticle according to claim 1 or 2, **characterized in that** the polymer is chosen as being the polymethyl methacrylate or a polymethyl methacrylate/polycaprolactone combination in a polymethyl methacrylate/ polycaprolactone weight ratio comprised between 1 and 10.

4. Microparticle according to any of claims 1 to 3, **characterized in that** the stabilizing agent is chosen as being the hexadecyltrimethylammonium bromide or the benzethonium chloride.

5. Microparticle according to any of claims 1 to 4, **characterized in that** the stabilizing agent/polymer weight ratio is comprised between 0.005 and 0.1.

6. Microparticle according to any of claims 1 to 5, **characterized in that** the shell further comprises a co-stabilizing agent chosen among the hexadecyltrimethylammonium bromide, the benzethonium chloride, the polyvinyl alcohol and the amino-dextran, said co-stabilizing agent being different from the stabilizing agent.

7. Microparticle according to any of claims 1 to 6, **characterized in that** its zeta potential is greater than or equal to 30 mV.

8. Microparticle according to any of claims 1 to 7, **characterized in that** it contains a lipophilic or hydrophilic active agent.

9. Process for preparing microparticles according to claim 8 comprising the following steps:
- preparing an organic solution by dissolving the polymer previously described and the lipophilic active agent in an organic solvent;
- preparing an aqueous solution containing the stabilizing agent as defined previously;
- contacting the organic solution and the aqueous solution prepared accordingly under stirring for the time necessary to the formation of the emulsion;
- diluting the emulsion obtained accordingly in a large volume of water until complete evaporation of the organic solvent.

10. Process for preparing microparticles according to claim 8 comprising the following steps:
- preparing an organic solution containing the monomer(s) necessary for the preparation of the polymer previously described and the lipophilic active agent;
- preparing an aqueous solution containing the stabilizing agent as previously defined and a polymerization initiator;
- contacting the organic solution and the aqueous solution prepared accordingly under stirring for the time necessary to the formation of the emulsion.

11. Process for preparing microparticles according to claim 8 comprising the following steps:
- preparing an aqueous solution (1) by solubilizing the hydrophilic active agent in an aqueous solution containing a stabilizing gent as previously defined;
- preparing an aqueous solution (2) containing a stabilizing agent as previously defined, said stabilizing agent being identical to or different from the one contained in the aqueous solution (1);
- preparing an organic solution by dissolving the polymer previously described in an organic solvent such as the dichloromethane, the ethyl acetate or the acetone;
- contacting the aqueous solution (1) and the organic solution prepared accordingly under vigorous stirring for the time necessary to the formation of an emulsion (1).
- contacting the emulsion (1) prepared accordingly with the aqueous solution (2) under vigorous stirring for the time necessary to the formation of the emulsion (2);
- diluting the emulsion (2) obtained accordingly in a large volume of water until complete evaporation of the organic solvent.

12. Use of microparticles according to claim 8 for preparing a cosmetotextile.

13. Textile article comprising at least one microparticle according to claim 8.

14. Solution comprising at least one microparticle according to claim 8.

15. Powder comprising a microparticle according to claim 8.

16. Tablet comprising a microparticle according to claim 8.
